# EUROPÄISCHE PATENTSCHRIFT

(11) **EP 1 784 201 B1**
(45) Veröffentlichungstag und Bekanntmachung des Hinweises auf die Patenterteilung: **20.10.2010**
(21) Anmeldenummer: 05776027.4
(22) Anmeldetag: 21.07.2005
(51) Int. Cl.: A61K 36/282, A61K 31/352, A61K 31/121, A61P 5/48, A61K 31/09, A61P 3/10, A61K 45/06

(54) **PHYSIOLOGISCH AKTIVE ZUSAMMENSETZUNG GEGEN DIABETES**
PHYSIOLOGICALLY ACTIVE COMPOSITION AGAINST DIABETES
COMPOSITION PHYSIOLOGIQUEMENT ACTIVE CONTRE LE DIABETE

(30) Priorität: 24.07.2004 DE 102004036047
(43) Veröffentlichungstag der Anmeldung: 16.05.2007
(73) Patentinhaber: Phytomedics Incorporated, Dyton, NJ 08810 (US)
(72) Erfinder: JÄGER, Ralf, Milwaukee, WI 53202 (US); WENK, Hans-Henning, D-45470 Mülheim a.d. Ruhr (DE); DIECK, Heike, Tom, D-85354 Freising (DE); HOPPE, Hans, Ullrich, 85416 Lagenbach (DE); RABELER, Roland, 85356 Freising (DE)
(74) Vertreter: Böhm, Brigitte
(86) Internationale Anmeldenummer: PCT/EP2005/007964
(87) Internationale Veröffentlichungsnummer: WO 2006/010560

(56) Entgegenhaltungen:
- EP-A- 0 183 436
- WO-A-99/38479
- WO-A-03/020026
- MADUREIRA ET AL: "A new sesquiterpene-coumarin ether and a new abietane diterpene and their effects as inhibitors of P-glycoprotein" PLANTA MEDICA, Bd. 26, Nr. 6, Dezember 2004 (2004-12), XP018001499
- MARES D ET AL: "ANTIDERMATOPHYTIC ACTIVITY OF HERNIARIN IN PREPARATION OF CHAMOMILLA RECUTITA (L.) RAUSCHERT" PLANTES MEDICINALES ET PHYTOTHERAPIE, ANGERS, FR, Bd. 26, 1993, Seiten 91-100, XP000917217 ISSN: 0032-0994
- JAYASINGHE L ET AL: "Antifungal constituents of the stem bark of Bridelia retusa" PHYTOCHEMISTRY, PERGAMON PRESS, GB, Bd. 62, Nr. 4, Februar 2003 (2003-02), Seiten 637-641, XP004404091 ISSN: 0031-9422
- MALLAVARAPU G R ET AL: "COMPOSITION OF THE ESSENTIAL OIL OF CYMBOPOGON-TRAVANCORENSIS" PLANTA MEDICA, Bd. 58, Nr. 2, 1992, Seiten 219-220, XP008061012 ISSN: 0032-0943
- XIAO Y-Q ET AL: "STUDIES ON THE CHEMICAL CONSTITUENTS OF ARTEMISIA-ANOMALA" ACTA BOTANICA SINICA, Bd. 28, Nr. 3, 1986, Seiten 307-310, XP008061015 ISSN: 0577-7496
- KOMIYA T ET AL: "STUDIES ON INCHINKO PART 2 STUDIES ON THE COMPOUNDS RELATED TO CAPILLARISIN AND FLAVONOIDS" YAKUGAKU ZASSHI, Bd. 96, Nr. 7, 1976, Seiten 855-862, XP008061013 ISSN: 0031-6903
- OKADA Y ET AL: "Search for naturally occuring substances to prevent the complications of diabetes. II. Inhibitory effect of coumarin and flavonoid derivatives on bovine lens aldose reductase and rabbit platelet aggregation" CHEMICAL AND PHARMACEUTICAL BULLETIN, PHARMACEUTICAL SOCIETY OF JAPAN, TOKYO, JP, Bd. 43, Nr. 8, August 1995 (1995-08), Seiten 1385-1387, XP002958799 ISSN: 0009-2363
- FONSECA F N ET AL: "Critical assessment of electrolyte systems for the capillary electrophoresis analysis of phenolic compounds in herbal extracts" JOURNAL OF MICROCOLUMN SEPARATIONS, PROVO, UT, US, Bd. 13, Nr. 6, 2001, Seiten 227-235, XP002961061

## Beschreibung

Gegenstand der vorliegenden Erfindung ist eine physiologisch aktive Zusammensetzung enthaltene effektive Mengen definierter Verbindungen sowie deren Verwendung. gemäss der Ansprüche.

Zusammensetzungen, die natürliche Extrakte bzw. darin enthaltene Hauptwirkstoffe enthalten, werden vom Verbraucher immer häufiger beider Selbstmedikation von Stoffwechselerkrankungen eingesetzt. Dabei gilt das Vertrauen insbesondere Extrakten auf pflanzlicher Basis, die bspw. aus der Naturheilkunde bekannt sind oder die auf nachgewiesene Effekte in fremden Kulturkreisen zurückgehen.

Als typische Volkskrankheiten gelten Herz-Kreislauferkrankungen sowie Stoffwechselerkrankungen, wie insbesondere Diabetes und dessen Vorformen sowie krankhaft veränderte Blutwerte, die auf eine unausgewogene Ernährung bzw. mangelnde Bewegung zurückzuführen sind, wobei insbesondere gesteigerte Cholesterin- oder Blutfettwerte zu nennen sind.

Insbesondere im Zusammenhang mit Stoffwechselerkrankungen sind zwischenzeitlich zahlreiche Präparate rezeptfrei im Markt verfügbar, wobei das zunehmende Interesse der Vorbeugung und Behandlung von Diabetes und insbesondere Diabetes Typ 2 gilt.

Aus dem Stand der Technik sind mehrere Dokumente bekannt, die sich mit der Behandlung von Diabetes mit Hilfe natürlicher Extrakte oder Pflanzeninhaltsstoffe befassen.

So beschreibt die internationale Patentanmeldung WO 00/15174 A2 die Verwendung von Bioflavonoiden definierter Zusammensetzung auch in Form eines Pflanzenextraktes, zur Senkung des Blutglukosespiegels bei Säugern. Als typische Bioflavonoide sind Hesperidin, Hesperetin, Naringin, Naringenin, Diosmin, Rutin und Quercetin genannt.

Die europäische Patentanmeldung EP 0 902 870 A1 offenbart ebenfalls u.a. Naringenin und Naringin als geeignete Flavanone zur Erniedrigung des Blutglukosespiegels oder des Fettspiegels, aber auch zur Prävention von Diabetes und Hyperlipidämie. In diesem Zusammenhang ist auch offenbart, dass Flavanone aus Pflanzenextrakten, wie z.B. aus Zitrusfrüchten, stammen können.

Gemäß der Veröffentlichung in Herba Hungarica 1987, Tom. 26, Nr. 1 können Flavonoide, wie sie in unterschiedlichen Organen ausgewählter Bauhinia-Spezies enthalten sind, einen Einfluss auf den Blutglukosespiegel zeigen. In diesem Zusammenhang werden insbesondere Quercitroside und Kaempferol-3-galactoside sowie -rhamnoglucoside genannt; aber auch 5,7-Dimethoxyflavanone sowie 4-O-L-Rhamnopyranosil-β-D-glycocyranoside sind erwähnt.

Schließlich beansprucht die internationale Patentanmeldung WO 03/020026 A1 unterschiedliche Verfahren zur Beeinflussung des Blutglukosespiegels, der Aktivität des Glukagon-ähnlichen Peptids 1 (GLP1), der Insulin-abhängigen Glukoseaufnahme oder aber auch Verfahren zur Behandlung von Typ 2 Diabetes, wobei allgemein effektive Dosen eines mäßig polaren Extraktes von Artemisia-Spezies und insbesondere von Artemisia dracunculus eingenommen werden soll.

Unter Diabetes versteht man allgemein ein komplexes Stoffwechselbild bzw. eine Krankheit, die in den meisten Fällen durch erhöhte Blutzuckerspiegel charakterisiert ist, womit auch schwerwiegende Einflüsse auf den Metabolismus von Kohlenhydraten, Fetten und Proteinen einhergehen. Dieses Krankheitsbild resultiert aus der Unfähigkeit, den Blutzuckerspiegel zu kontrollieren, was bspw. auf einen ungenügenden Insulinspiegel (absoluter Insulinmangel) oder eine nicht ausreichende Insulinaktivität (relativer Insulinmangel) zurückgeht. Erhöhte Glukosespiegel wiederum führen zu sekundären Gesundheitsproblemen, die zusätzliche Behandlungsschritte notwendig machen. Als Hauptrisikofaktoren, welche mit Diabetes assoziiert sind, sind Arteriosklerose, diabetische Retinopathie, Katarakt, Nephropathien, erhöhte Infektionsgefahren, Bluthochdruck, Nervenerkrankungen, aber auch Demenz zu sehen.

Insgesamt unterscheidet man zwischen zwei Hauptdiabetestypen mit zahlreichen Variationen. Typ 1 Diabetes betrifft allgemein Kinder oder Jugendliche und resultiert aus der Unfähigkeit des Körpers, Insulin zu produzieren (absoluter Insulinmangel). Typ 2 Diabetes, der Typ 1 Diabetes hinsichtlich seiner Häufigkeit bei weitem übersteigt, basiert entweder auf einer verringerten Insulinsekretion oder häufiger auf einer Insulinresistenz (relativer Insulinmangel). Die üblicherweise angewendeten Behandlungsmethoden sind darauf ausgerichtet, den Blutzuckerspiegel zu senken, indem die nicht-selektive Glukoseaufnahme in Zellen gefördert wird. Diese wenig kontrollierte Glukoseaufnahme betrifft allerdings auch die so genannten Adipozyten, wodurch wiederum ein Gewichtsproblem entstehen kann. Wichtig ist in diesen Fällen deshalb die strenge Kontrolle der Behandlungsmethode und der damit erzielten Veränderungen. Die verbreitetste herkömmliche Behandlungsmethode, nämlich die Verabreichung von Insulin, ist nach wie vor sehr teuer und für den Patienten auch immer noch mit Unannehmlichkeiten und Nebenwirkungen wie der Gefahr einer Hypoglykämie durch Überdosierung, allergischen Reaktionen sowie Entwicklung einer lokalen Lipodystrophie an den Injektionsstellen verbunden.

Den konventionellen Behandlungsmethoden stehen sogenannte OTC-Medikationen ("Over-the-counter") entgegen, wie sie die bereits erwähnten natürlichen Produkte, insbesondere auf pflanzlicher Basis, darstellen. Eine der zahlreichen Pflanzenfamilien, die in der traditionellen Naturheilung eingesetzt werden, ist die Artemisia-Familie mit über 400 Spezies. Von Artemisia dracunculus wurde bspw. von Yazdanparast et al in Biomedical Letters 59 (1999), Seiten 137 bis 141, berichtet, dass Alkohol-basierte Extrakte davon in der Lage sind, antihyperlipämische Effekte auf Ratten auszuüben. Wie in der bereits erwähnten PCT-Anmeldung WO 03/020026 ausführlich .dargelegt, werden aber auch von anderen Artemisia-Spezies, wie bspw. A. herba-alba oder A. judaica, positive Effekte im Zusammenhang mit einem gestörten Fett- oder Zuckerhaushalt berichtet.

Das generelle Problem bei natürlichen Extrakten liegt in der nur schwer durchzuführenden Standardisierung, zumal sowohl die Anbau- und Erntebedingungen, als auch Lagerkonditionen und die Art der Aufarbeitung signifikante Einflüsse auf die Inhaltsstoffe und deren jeweiligen Anteil im Ausgangsmaterial sowie im daraus erhaltenen Extrakt haben. Bekanntermaßen wirken sich die Herkunft und insbesondere die damit verbundenen Klimafaktoren und die Bodenqualität deutlich auf Qualität und Quantität der Pflanzeninhaltsstoffe aus. Aber auch Einflüsse wie Lagertemperatur, Luft- und Feuchtigkeitseinwirkung auf das bereits geerntete Material spielen bspw. in Form von oxidativen Einflüssen eine Rolle. Hinzu kommt natürlich die Art der Aufarbeitung und insbesondere die Art und Qualität der verwendeten Lösemittel, so dass selbst bei identischen Pflanzenspezies gravierende Unterschiede in der Zusammensetzung von Pflanzenextrakten festzustellen sind.

Dies kommt insbesondere bei den bereits beschriebenen Artemisia-Varietäten zum Tragen, bei denen es sich um Vertreter von Estragon handelt. Der sogenannte "deutsche" oder "französische" Estragon stellt die am stärksten aromatische Zuchtform dar und enthält bis zu 3 % ätherisches Öl, dessen Aroma von Methylchavicol (Estragol) und -eugenol beherrscht wird. Enthalten sind ferner p-Methoxyzimtsäure, Phellandren, α- und β-Pinen, Camphen, Ocimen und Limonen: Eine andere Sorte, nämlich der "russische" Estragon, enthält wesentlich weniger ätherische Öle, wobei Estragol vollständig fehlt, wodurch diese Varietät nicht das sonst so angenehme süßliche Aroma des französischen Estragons entwickelt. Die stattdessen enthaltenen Flavonoide Quercetin und Patuletin zeichnen sich durch einen herben und adstringierenden Geschmack aus. Russischer Estragon wird deshalb üblicherweise nicht als typisches Küchenkraut bezeichnet, sondern eher den ursprünglichen Wildformen des Estragons zugeordnet. Russischer Estragon jedoch lässt sich in kälterem Klima leichter kultivieren: Eine Übersicht über potentielle Inhaltsstoffe von Artemisia-Varietäten und insbesondere Artemisia dracunculus ist der Übersicht von Agricultural Research Service ("Phytochemical and Ethnobotanical Databases") zu entnehmen (http://sun.arsgrm.gov:8080/npgspub/xsq!/duke/plantdisp.xsql?taxon=123).

Auch in der bereits mehrfach zitierten internationalen Patentanmeldung WO 03/020026 A1 sind typische Vertreter von Inhaltsstoffen aufgeführt, die auch in Artemisia-Extrakten enthalten sein können. So werden exemplarisch Capillarisin, Tetrahydroxy-Methoxy-Flavanone, Umbelliferone, Sakuranin, Trihydroxy-Methoxy-Flavanone und Trihydroxy-Flavanone aufgeführt. Dieses Dokument offenbart auch verschiedene Chromatogramme von frischen und gefrorenen Artemisia-Extrakten, wobei die jeweils angegebenen Peaks allerdings keine Rückschlüsse auf möglicherweise enthaltene definierte Verbindungen zulassen, obwohl in den Beispielen Estragol und Methyleugenol als typische Inhaltsstoffe von Artemisia dracunculus-Extrakten genannt sind. Aufgrund der ebenfalls gemachten Aussage, dass sich bei ausgewachsenen Pflanzen das Biomasseverhältnis von den Blättern zum Stängel hin verschiebt, wurden demzufolge die gemäß diesem Dokument untersuchten Extrakte hauptsächlich an getrennten Stängeln und Blättern vorgenommen. Die bevorzugten Extraktprofile wurden an aufgearbeiteten Artemisia-Blättern erstellt, wobei lediglich eine relative Beurteilung der näher untersuchten Extrakte vorgenommen worden ist. So ist bspw. erwähnt, dass Extrakte von Artemisia dracunculus, die aus gefrorenem Pflanzenmaterial gewonnen wurden, eine größere Aktivität zeigen als frisches Pflanzenmaterial. Lediglich anhand eines Vergleiches mit dem Wiley-Verzeichnis von Massenspektren konnten Haupt-Peaks identifiziert werden, was offensichtlich zu den oben genannten Verbindungen als potentielle Inhaltsstoffe geführt hat. Weitere Inhaltsstoffe, die u.a. für die mitbeanspruchte positive Wirkung auf einen gestörten Blutzuckerspiegel verantwortlich sein können, wurden allerdings in den beschriebenen, schwach-polaren ethanolischen Extrakten nicht identifiziert.

Eine Aufgabe der vorliegenden Erfindung war es deshalb, eine physiologisch aktive Zusammensetzung bereitzustellen, mit welcher diese im Stand der Technik auftretenden Schwierigkeiten überwunden werden können, und die insbesondere aus definierten Verbindungen oder Verbindungsklassen besteht und in effektiven Dosen auch für die oben beschriebenen Syndrome und Symptome angewendet werden kann. Diese Zusammensetzung sollte möglichst in leicht zugänglichem Pflanzenmaterial enthalten und in standardisierter Form leicht herzustellen sein. Außerdem sollte diese Zusammensetzung aus Komponenten bestehen, die über die aus dem Stand der Technik bekannten Verbindungen hinausgehen und im Zusammenwirken mit diesen, aber auch untereinander, nach Möglichkeit synergistische Effekte im Hinblick auf das jeweils gewählte Anwendungsgebiet aufweisen.

Erfindungsgemäß gelöst wurde diese Aufgabe mit einer entsprechenden Zusammensetzung, die effektive Mengen mindestens einer Verbindung, ausgewählt aus 4-O-Methyl-davidigenin, 4'-O-Methyl-davidigenin, Davidigenin, Elemicin, Isoelemicin, Herniarin, Demethoxycapillarisin, insbesondere 6-Demethoxycapillarisin und/oder 6-Demethoxy-3'-Methoxycapillarisin, Hispiludin und 9-Hydroxy-10E, 12Z, 15Z-octadecatriensäure sowie deren Glykoside, nämlich Glucoside und/oder Rhamnoglucoside von 4-O-Methyldavidigenin und/oder 4'-O-Methyldavidigenin, umfasst. Dabei können auch deren jeweilige geeignete Salze umfasst sein sowie alle Verbindungen, die mit Hilfe der HPLC-Analyse in einem Artemisia-Extrakt detektierbar sind.

Überraschend wurde festgestellt, dass die beanspruchten Verbindungen tatsächlich in Pflanzenextrakten nachgewiesen werden konnten, welche als Grundlage für die Herstellung von Mitteln zur Behandlung von Stoffwechselerkrankungen eingesetzt werden. Im Gegensatz zu den bisher bekannten Veröffentlichungen konnten diese Verbindungen vorzugsweise in Artemisia-Extrakten festgestellt werden, wobei entgegen den Erwartungen die jeweiligen Konzentrationen in einer Menge auftraten, wie sie aus den bisher bekannten Analysen nicht ableitbar sind. Als weiterer Vorteil wurde festgestellt, dass die beanspruchte Zusammensetzung insbesondere dadurch leicht zugänglich ist, dass sie nicht nur durch Kombination der Reinsubstanzen erhältlich ist, sondern in einfacher Weise auch durch die Auswahl geeigneter Pflanzenextrakte, wobei die Herstellung dieser Pflanzenextrakte selbst nicht über die bislang bekannten Aufarbeitungsverfahren von Pflanzenmateüal hinausgehen muss.

Die beanspruchte Zusammensetzung zeichnet sich auch dadurch aus, dass sie zusätzlich mindestens eine Verbindung, ausgewählt aus Chlorogensäure, Naringenin, Flavonoiden, Flavanoiden, Terpenen, Terpenoiden umfassen, . Diese Verbindungen sind als Einzelsubstanzen zwar nicht neu, können aber in Verbindung mit den erfindungsgemäß umfassten Komponenten auch synergistische Effekte, bevorzugt additive Wirkungen, entfalten.

Gemäß der vorliegenden Erfindung wird eine Zusammensetzung bevorzugt, die die Verbindungen in reiner Form und/oder als Mischung umfasst, wobei die Mischung dann vorzugsweise als natürlicher Extrakt vorliegt. Aufgrund ihrer physikalisch/chemischen Eigenschaften sind die von der erfindungsgemäßen Zusammensetzung umfassten Verbindungen insbesondere als alkoholische Extrakte hinsichtlich ihrer Zubereitungsform geeignet, wobei besonders bevorzugt alkoholisch/wässrige Extrakte und in diesem Zusammenhang insbesondere deren Ether-Fraktionen geeignet sind.

Alkohole als bekanntermaßen polare Flüssigkeiten und vor allen Dingen Methanol, Ethanol und Isopropanol eignen sich hervorragend zur Herstellung derartiger Extrakte, die im vorliegenden Fall als besonderes Kennzeichen keinerlei mutagene Aktivitäten entfalten oder aber bedenkliche Toxine enthalten.

Die Herstellung derartiger Extrakte ist in keiner Weise limitiert, jedoch empfiehlt es sich, das pflanzliche Ausgangsmaterial zunächst mit einer sogenannten Induktor-Komponente in Kontakt zu bringen, wofür sich insbesondere Polysaccharide, wie bspw. Chitosan, aber auch Essigsäure, Methylsalicylat, Methyljasmonat oder sogar Mikroorganismen besonders eignen. Anschließend wird das so aufgeschlossene Ausgangsmaterial mit der Extraktionsflüssigkeit, bspw. der alkoholisch/wässrigen Lösung, behandelt. Dabei sollte der Alkohol-Gehalt zwischen 40 und 75 % liegen, wobei sich bevorzugt Alkohol-Gehalte, davon insbesondere Ethanol-Gehalte, zwischen 50 und 60 % als sehr geeignet erwiesen haben.

Zusätzlich und in Abhängigkeit vom gewählten Ausgangsmaterial kann dieses auch durch mechanische Maßnahmen vorbehandelt oder während des Extraktionsvorgangs zusätzlich aufbereitet werden, wodurch insbesondere die Zellwand und Membranstrukturen zerstört und somit die Inhaltsstoffe freigesetzt und dem Lösemittel zugänglich gemacht werden. Hierfür sollten insbesondere übliche Vermahlprozesse gewählt werden. Es ist allerdings auch möglich, die Trocknung des frischen oder gefrorenen und wieder aufgetauten Ausgangsmaterials bei erhöhten Temperaturen durchzuführen, um so die Konzentration von Methyleugenol zu senken, welches einen Inhaltstoff darstellt, bei dem es sich um ein anerkanntes Karzinogen handelt und welcher insbesondere in bestimmten Asteraceen, wie sie auch Estragon-Pflanzen darstellen, vorkommt.

Von der vorliegenden Erfindung werden insbesondere Zusammensetzungen als bevorzugt umfasst, bei denen es sich um einen Artemisia-Extrakt handelt. Hierbei sind Extrakte von Artemisia dracunculus, A. herba-alba, A. judaica, A. vulgaris, A. abysinica, A. absynthicum, A. afra, A. cannariensis, A. pallens, A. annua, A. abrotanum, A. ludoviciana, A. capillaris und A. scoparia besonders geeignet.

Bei der Analyse der in diesen Extrakten enthaltenen Inhaltsstoffe haben sich als besonders geeignete Subspezies von A. dracunculus insbesondere solche von russischem oder französischem Estragon als besonders vorteilhaft erwiesen, da diese die als erfindungswesentlich enthaltenen Verbindungen in ausreichenden und stabilen Konzentrationen enthalten.

In diesem Zusammenhang sei auch darauf verwiesen, dass die erfindungsgemäße Zusammensetzung sich vorteilhafterweise dadurch auszeichnet, dass sie die jeweilige Verbindung bzw. Mischung oder den Extrakt in effektiven Mengen enthält, die zwischen 1 und 1,000,000 ppm liegen, wobei der maximale Wert die Reinsubstanz kennzeichnet. Bevorzugt werden allerdings effektive Mengen zwischen 10 und 20,000 ppm und besonders bevorzugt zwischen 100 und 5,000 ppm. Diese Mengen spiegeln insbesondere die Tatsache wider, dass die unterschiedlichen Verbindungen ihre zum Teil voreinander abweichenden, sich aber ergänzenden Wirkungen in unterschiedlichen Mengen entfalten, die, wie bereits dargelegt, vom annähernd homöopathischen Bereich bis zur Reinsubstanz reichen können.

Die vorliegende Erfindung deckt die Verwendung dieser Zusammensetzungen zur Herstellung eines Mittels zur Vorbeugung und/oder Behandlung von (Prä)-Diabetes und damit zusammenhängenden Formen, Begleit-und/oder Folgeerkrankungen ab, wobei mit solchem Mittel, der Blutzuckerspiegel bei Säugern, die Insulinresistenz, die Expression des IRS-2 ("Insulin-Rezeptor-Substrat 2) Polypeptides die Insulin-gesteuerte Glukoseaufnahme und/oder der postprandiale Glucose-Spiegel beeinflusst werden. Geeignet ist aber auch ein Mittel, das zur Behandlung oder Vorbeugung von Diabetes Typ 2 und/oder 1, zur Behandlung oder Vorbeugung von Prä-Diabetes und : zur gezielten Beeinflussung des Körpergewichtes und/oder zur Steigerung der körperlichen Leistungsfähigkeit herangezogen werden kann.

Die genannten Möglichkeiten zur Beeinflussung von Stoffwechselvorgängen können im Falle der Beeinflussung des Blutzuckerspiegels, der Insulinresistenz, und/oder des postprandialen Glukose-Spiegels zur Senkung dienen und in den Fällen der Expression des IRS-2-Polypeptids sowie der Insulin-gesteuerten Glukoseaufnahme zu deren Erhöhung stattfinden.

In einer anderen Ausführungsform berücksichtigt die vorliegende Erfindung die Verwendung gemäss der Ansprüche des Mittels als Nahrungsergänzungsmittel, Getränk, (diätetisches) Lebensmittel und/oder Funktionsnahrungsmittel. Möglich ist allerdings auch dessen Einsatz im Rahmen der klinischen Ernährung und/oder als Sportlernahrung, wobei jeweils der therapeutische Anwendungsbereich im Vordergrund steht. Die mit der beanspruchten Zusammensetzung herstellbaren Mittel eignen sich somit insbesondere für die sogenannten OTC-Produkte und damit auch zur Selbstmedikation.

Unabhängig vom jeweiligen - Verwendungszweck und der damit verbundenen Applikationsform sieht die vorliegende Erfindung die Verwendung des Mittels in Mengen vor, bei denen die effektive Tagesmenge der Zusammensetzung zwischen 0,1 und 500 mg/kg Körpergewicht liegt, wobei Bereiche zwischen 1,5 und 150 mg/kg Körpergewicht als bevorzugt und 15 mg/kg Körpergewicht als besonders bevorzugt gelten.

Neben den erfindungsgemäß enthaltenen Verbindungen und den zusätzlich aufgeführten Verbindungen kann das Mittel auch noch mindestens eine aktive Verbindung und/oder einen aktiven Extrakt umfassen, die ausgewählt sind aus Gymnema sylvestre, Bockshornklee ("Fenugreek"), Bittermelone, α-Liponsäure (-Salze), Corosol-Säure, Ursolinsäure, D-Pinitol, Aloe vera, Chrompicolinat, Banaba, Yacou, Momordica charantia, Olive, Pherocarpus marsupium, Salacia reticulata, Knoblauch, Weißdorn, Phospholipiden und insbesondere Phosphatidylserin, Omega-3-Fettsäuren und Stärke. Das so zusammengesetzte Mittel kann im Rahmen der vorliegenden Erfindung insbesondere zur Vorbeugung und Behandlung von (Prä-)Diabetes eingesetzt werden.

Mitumfasst von der vorliegenden Erfindung ist aber auch die Verwendung eines Mittels, das neben der physiologisch aktiven Zusammensetzung mindestens eine aktive Verbindung, ausgewählt aus Brenztraubensäure, L-Carnitin, Hydroxycitronensäure, Ephedrin, Koffein, konjugierte Linolsäure, Acetylsalicylsäure, α-Liponsäure und/oder deren Salzen umfasst. Dieses Mittel hat sich als besonders geeignet im Hinblick auf die Körpergewichtskontrolle und insbesondere für die Senkung des Körpergewichts gezeigt.

Möglich im Rahmen der vorliegenden Erfindung ist aber auch die Verwendung eines Mittels, das neben der physiologisch aktiven Zusammensetzung mindestens eine aktive Verbindung, ausgewählt aus Guanidin-Verbindungen, insbesondere Kreatin, Kreatin-Monohydrat, Guanidinessigsäure, Kreatinol, Kreatin-Citronensäure-Verbindung, Kreatin-pyruvat, Phosphokreatin, sowie Koffein, α-Liponsäure, Glukosamin, Chondroitin, hydrolysiertes Kollagen, Methylsulfonyl-Methan, Molkeprotein, L-Glutamin, Phospholipiden, insbesondere Phosphatidylserin, Phosphatidylcholin, sowie Cholin, β-Hydroxy-β-methylbutyrat, Brenztraubensäure, L-Carnitin, D-Ribose, Aminosäuren, S-Adenosylmethionin, Taurin, konjugierte Linolsäure, Glycerin, Zimt und/oder Salze umfasst. Diese Variante des Mittels ist vor allen Dingen zur Steigerung der Zellenergie in nicht-adiposen Zellen geeignet.

Weiterhin wird gemäß der vorliegenden Erfindung ein Arzneimittel beansprucht, welches die erfindungsgemäße Zusammensetzung allein oder in Kombination mit mindestens einer der oben genannten aktiven Verbindungen und/oder Extrakte umfasst, wie beansprucht.

Insgesamt konnte mit der beanspruchten Zusammensetzung und den damit verbundenen speziellen Anwendungsfällen nicht nur die Aufgabenstellung voll erfüllt werden, sondern es konnten insbesondere definierte Verbindungen mit einem positiven Effekt auf Stoffwechselprozesse in Verbindung gebracht werden, wobei diese Verbindungen sowohl als Reinsubstanz, aber auch in Form von Extrakten und insbesondere pflanzlichen Extrakten die gewünschten Wirkungen entfalten. Dabei kann die Wirksamkeit dieser Verbindungen durch Kombination untereinander, aber auch mit anderen Verbindungsklassen zum Teil überadditiv gesteigert werden, was insbesondere im Zusammenhang mit der Selbstmedikation im nicht-medizinischen Bereich für den Endverbraucher deutliche Vorteile bringt.

Die nachfolgenden Beispiele veranschaulichen die genannten Vorzüge der vorliegenden Erfindung.

### Beispiele

### Beispiel 1: Herstellung eines Estragon-Extraktes

500 g getrocknete oberirdische Pflanzenteile von russischem Estragon (*Artemisia dracunculus*) wurden zerkleinert ( Teilchengröße < 10 mm) und mit 81 Ethanol 80% v/v 16 h lang bei 45 °C extrahiert. Nachfolgend wurde der Extrakt filtriert, das Filtrat am Rotationsverdampfer auf 200 ml konzentriert, mit 50 g mikrokristalliner Cellulose versetzt und gefriergetrocknet. Es wurden 175 g eines grünlichen Pulvers erhalten.

HPLC-Analyse des Extraktes ergab die folgenden Gehalte:

| | |
|---|---|
| Herniarin | 360 ppm |
| Davidigenin | 990 ppm |
| 6-Demethoxycapillarisin | 1450 ppm |
| 6-Demethoxy-3'-Methoxycapillarisin | 15 ppm |
| Elemicin | 195 ppm |
| Isoelemicin | 420 ppm |
| Hispiludin | 65 ppm |
| 9-Hydroxy-10E,12Z,15Z-octadecatriensäure | 25 ppm |
| 4'-O-Methyldavidigenin | 465 ppm |
| 4-O-Methyldavidigenin | 35 ppm |
| 4-O-Methyldavidigenin-4'-glucosid | 585 ppm |
| 4-O-Methyldavicligenin-4'-rhamnoglucosid | 675 ppm |

### Beispiel 2: Akute Effekte des alkoholischen Estragon-Extraktes auf den postprandialen Blutglukosespiegel in Diabetes Typ 2-Tiermodellen.

Nachfolgend sind die Ergebnisse der Tierstudien aufgeführt, bei der die akute Wirkung eines Estragon-Extraktes bei Tieren mit Adipositas und gleichzeitig erhöhtem Nüchtern-Blutzucker (Diabetes Typ 2) auf den postprandialen Blutglucosespiegel untersucht wurde. Eingesetzt wurde eine Glukose-Lösung, die ein gemäß Beispiel 1 erhaltenes Pulver enthielt.

Die Untersuchungsdauer betrug jeweils max. 5 Stunden, wobei mit jedem Tier entweder mit verschiedenen Dosen des Extraktes oder mit Placebo ein oraler Glucosetoleranztest durchgeführt wurde.

Insgesamt wurden in der Studie 30 Ratten mit erhöhtem Blutzuckerspiegel bzw. ob/ob-Mäuse (adipöse Mäuse, die homozygot für das Gen ob (nach englisch obese = fettleibig) sind) untersucht, wobei je Behandlungsgruppe 10 Tiere untersucht wurden. Nach 14-stündiger Fastenperiode wurde bei den Tieren der Nüchternblutzuckerspiegel bestimmt. Er betrug durchschnittlich 138,8 mg/dl (Mäuse) bzw. 136,0 mg/dl Glukose (Ratten) und unterschied sich zwischen den Gruppen nicht signifikant. Den Tieren wurde anschließend eine definierte Menge Glukose in Lösung (3,33 ml Lösung/kg KG Ratte oder 10 ml/kg KG Maus) mittels Schlundsondenfütterung verabreicht, die entweder 500 mg/kg KG oder 1000 mg/kg KG der Extrakt-Lösung enthielt bzw. zur Kontrolle nur aus der Glukoselösung bestand (60%ig (Ratte) bzw. 20%ig (Mäuse) + 2 % Tween 80 als Lösungsvermittler). Nach Verabreichung der Glukoselösung wurde in 15 Minuten-Abständen über einen Zeitraum von 180 min Blut zur Bestimmung der Blutglucosekonzentration und der Insulinkonzentration entnommen. Die Werte der postprandialen Glucosespiegel sind den Tabellen 1 und 2 zu entnehmen.

Alle Tiere vertrugen die Behandlungen ohne irgendwelche Anzeichen von Unverträglichkeit.

**Tabelle 1: postprandiale Blutglucosekonzentrationen (in mg/dl) bei diabetischen Mäusen, die mit 500 mg/kg KG oder 1000 mg/kg KG Extrakt in Glukoselösung bzw. Kontrollglukoselösung behandelt wurden.**

| Zeit [min] | 0 | 15 | 30 | 60 | 90 | 120 | 180 |
|---|---|---|---|---|---|---|---|
| | Kontrolle | | | | | | |
| Mittelwert | 142,2 | 433,2 | 686,0 | 357,1 | 340,4 | 335,0 | 313,5 |
| SD (Standard-abweichung) | 54,2 | 88,2 | 118,9 | 110,5 | 124,2 | 131,9 | 97,5 |
| | 500 mg/kg KG | | | | | | |
| Mittelwert | 143,7 | 353,9 | 534,2 | 319,7 | 274,0 | 260,5 | 259,9 |
| SD | 60,4 | 85,7 | 100,2 | 71,8 | 77,9 | 61,5 | 50,7 |
| | 1000 mg/kg KG | | | | | | |
| Mittelwert | 130,5 | 313,4 | 476,9 | 300,9 | 265,6 | 251,3 | 236,1 |
| SD | 44,6 | 88,1 | 100,9 | 76,8 | 63,8 | 75,4 | 76,6 |

**Tabelle 2: postprandiale Blutglucosekonzentrationen (in mg/dl) bei diabetischen Ratten, die mit 500 mg/kg KG 1000 mg/kg KG Extrakt in Glukoselösung bzw. Kontrollglukoselösung behandelt wurden.**

| Zeit [min] | 0 | 15 | 30 | 60 | 90 | 120 | 180 |
|---|---|---|---|---|---|---|---|
| | Kontrolle | | | | | | |
| Mittelwert | 133,2 | 323,7 | 430,2 | 452,6 | 367,5 | 286,3 | 235 |
| SD | 28,3 | 43,8 | 62.3 | 75.9 | 79.3 | 81,7 | 65,5 |
| | 500 mg/kg KG | | | | | | |
| Mittelwert | 135,2 | 306,9 | 382,4 | 364,2 | 294,8 | 230,5 | 195,8 |
| SD | 16,1 | 65,8 | 77,2 | 59,3 | 58,2 | 55,2 | 46,8 |
| | 1000 mg/kg KG | | | | | | |
| Mittelwert | 139,6 | 281,4 | 314,3 | 297,7 | 245,6 | 210,4 | 194,2 |
| SD | 42,2 | 99,8 | 82,4 | 91,8 | 102,2 | 93,2 | 55,4 |

### Beispiel 3: Akute Effekte des Estragon-Extrakts auf den postprandialen Blutglucosespiegel bei Personen mit Glucoseintoleranz bzw. Typ 2-Diabetes

Monozentrische, doppelblinde, randomisierte, placebo-kontrollierte cross-over Studie zur Untersuchung der akuten Wirksamkeit des Estragon-Extraktes auf den postprandialen Blutzuckerspiegel bei Personen mit Glucoseintoleranz bzw. Typ 2-Diabetes. Die Wirkung der Zubereitung wurde mittels eines Mahlzeitentoleranztestes (MTT; Meal tolerance test) ermittelt.

8 männliche oder weibliche, ausschließlich mit Diät behandelte Patienten mit erhöhten Nüchternblutzuckerspiegeln wurden in die Studie mit einbezogen. Die Studie umfasste neben einer Screening-Untersuchung (V0), bei der die möglichen Studienteilnehmer auf ihre Eignung zur Studienteilnahme hin untersucht werden, zwei ambulante Studientage Visite 1 (V1) + Visite 2 (V2), getrennt durch eine 2-bis 3- wöchige Wash-out-Periode sowie eine Abschlussuntersuchung im Anschluss an V2.

Nachdem die Eignung der Studienteilnehmer festgestellt wurde, wurden die Studienteilnehmer per Randomisierung einem Behandlungsplan zugeteilt und erhielten doppelblind im cross-over-Verfahren im Verlauf der Studie beide Behandlungen sowohl mit Extrakt als auch mit Plazebo (mikrokristalline Zellulose). An den jeweiligen Studientagen erhielten die Patienten jeweils vor einem standardisierten Frühstück eine einmalige orale Gabe von entweder 1000 mg Extrakt oder Placebo, wonach anschließend die Wirkung der Behandlung auf den postprandialen Blutzuckerspiegel durch regelmäßige Bestimmung der Blutzuckerwerte und anschließender Analyse der Serum-Insulinspiegel über einen Zeitraum von 5 Stunden erfasst wurde. Mit dem Abschluss von Visite 2, während deren Verlauf eine Abschlussuntersuchung durchgeführt wurde, war die Studie für die Studienteilnehmer beendet.

Voraussetzung für die Teilnahme eines Interessenten an einer klinischen Studie war seine schriftliche Einwilligung zur Teilnahme, nachdem er mündlich und schriftlich über Wesen, Bedeutung und Tragweite der klinischen Studie informiert wurde. Der Untersucher verpflichtete sich, die Studie in Übereinstimmung mit der Deklaration von Helsinki (in der Revision von Edinburgh, Oktober 2000), den Prinzipien der Good Clinical Practice (GCP), der International Conference of Harmonisation (ICH) und den nationalen Verordnungen und Richtlinien durchzuführen.

Die Studienteilnehmer wurden mit einem Informationsblatt über alle Aspekte der Studie sowie über den Datenschutz aufgeklärt. Die erfolgte Probandeninformation wurde zusätzlich zu der Einwilligungserklärung des Probanden durch die Unterschrift des verantwortlichen Prüfarztes dokumentiert.

Gemäß der Einschlusskriterien wurden 5 männliche und 3 weibliche Probanden im Alter zwischen 35 und 70 Jahren, einem BMI > 29 und < 40 kg/m², einem Nüchtern-Blutzucker ≥ 125 und ≤ 220 mg/dl, einem HbA1c-Wert > 6.1 % ohne antidiabetische Behandlung mit Tabletten oder Insulin sowie dem Vorliegen einer schriftlichen Einverständniserklärung in die Studie eingeschlossen. Diese Probanden wiesen nach Einschätzung des Prüfarztes keine klinisch signifikante Abweichung der Laborwerte in der Voruntersuchung auf (insbesondere: Serum-Kreatinin- und Serum-Hämoglobin-Werte, sowie Aktivität der Glutamat-Oxalacetat-Transaminase (GOT) und der Glutamat-Pyruvat-Transaminase (GPT), die auf eine akute oder chronische Erkrankung/Störung hindeuteten.

**Tabelle 3: postprandiale Plasmaglucosekonzentrationen (in mg/dl) bei diabetischen Probanden, behandelt mit 1000 mg Extrakt bzw. Plazebo zu einem Standardfrühstück**

| Zeit [min] | 0 1 | 30 | 60 | 90 | 120 | 180 | 300 |
|---|---|---|---|---|---|---|---|
| | Plazebo | | | | | | |
| Mittelwert | 142,4 | 202,7 | 234,4 | 243,7 | 223,5 | 185,7 | 152,6 |
| SD | 2,59 | 5,95 | 7,08 | 9,53 | 7,32 | 4,89 | 3,30 |
| | 1000 mg | | | | | | |
| Mittelwert | 141,3 | 185,7 | 218,4 | 207,6 | 184,8 | 159,3 | 133,7 |
| SD | 3,34 | 14,59 | 10,55 | 6,18 | 3,3 | 4,78 | 3,45 |

**Tabelle 4: postprandiale Plasmainsulinkonzentrationen (in pU/ml) bei diabetischen Probanden behandelt mit 1000 mg bzw. Plazebo zu einem Standardfrübstück**

| Zeit [min] | 0 | 30 | 60 | 90 | 120 | 180 | 300 |
|---|---|---|---|---|---|---|---|
| | Plazebo | | | | | | |
| Mittelwert | 14,1 | 79.9 | 149,8 | 173,8 | 178,7 | 148,3 | 56,9 |
| SD | 4,2 | 18,4 | 26,7 | 20,2 | 23,7 | 14,5 | 6,3 |
| | 1000 mg | | | | | | |
| Mittelwert | 13,2 | 72,1 | 134,4 | 156,7 | 145,5 | 118,6 | 42,9 |
| SD | 2,9 | 20,3 | 18,9 | 26,8 | 19,6 | 11,4 | 7,3 |

## Patentansprüche

1. Verwendung einer physiologisch aktiven Zusammensetzung umfassend effektive Mengen mindestens einer Verbindung, ausgewählt aus 4-O-Methyl-davidigenin, 4'-O-Methyl-davidigenin, Davidigenin, Elemicin, Isoelemicin, Herniarin, Demethoxycapillarisin, insbesondere 6-Demethoxycapillarisin und/oder 6-Demethoxy-3'-Methoxycapillarisin, Hispiludin und 9-Hydroxy-10E, 12Z, 15Z-octadecatriensäure, sowie deren Glykoside
und zusätzlich mindestens eine Verbindung, ausgewählt aus Chlorogensäure, Naringenin, Flavonoiden, Flavanoiden, Terpenen und/oder Terpenoiden
zur Herstellung eines Mittels zur Vorbeugung und/oder Behandlung von (Prä)-Diabetes und mit Diabetes zusammenhängenden Begleit- und/oder Folgeerkrankungen,
wobei die Glykoside ausgewählt sind aus der Gruppe bestehend aus Glucosiden und/oder Rhamnoglucosiden von 4-O-Methyldavidigenin und/oder 4'-O-Methyldavidigenin sowie deren Salze,
und wobei es sich bei der Vorbeugung und/oder Behandlung von (Prä)-Diabetes und mit Diabetes zusammenhängenden Begleit- und Folgeerkrankungen um eine Senkung
a) des Blutzuckerspiegels bei Säugern,
b) der Insulinresistenz,
c) des postprandialen Glucose-Spiegels,
und/oder um eine Erhöhung
d) der Expression des IRS-2 ("insulin-Rezeptor-Substrat-2")-Polypeptides und/oder
e) der Insulin-gesteuerten Glucoseaufnahme handelt.

2. Verwendung nach Anspruch 1,
**dadurch gekennzeichnet,**
**dass** sie mindestens eine Verbindung in Form von Glykosiden und/oder Aglyconen umfasst.

3. Verwendung nach Anspruch 1 oder 2,
**dadurch gekennzeichnet,**
**dass** sie die Verbindungen in reiner Form und/oder als Mischung umfasst.

4. Verwendung nach Anspruch 3,
**dadurch gekennzeichnet,**
**dass** sie die Mischung in Form eines natürlichen Extrakts umfasst.

5. Verwendung nach Anspruch 4,
**dadurch gekennzeichnet,**
**dass** sie einen alkoholischen und/oder einen alkoholisch/wässrigen Extrakt umfasst.

6. Verwendung nach Anspruch 5,
**dadurch gekennzeichnet,**
**dass** sie eine Etherfraktion des alkoholisch/wässrigen Extrakts umfasst.

7. Verwendung nach einem der Ansprüche 4 bis 6,
**dadurch gekennzeichnet,**
**dass** es sich um einen Artemisia-Extrakt handelt.

8. Verwendung nach Anspruch 7,
**dadurch gekennzeichnet,**
**dass** es sich bei dem Artemisia-Extrakt um einen Extrakt von A. dracunculus, A. herba-alba, A. judaica, A. vulgaris, A. abysinica, A. absynthicum, A. afra, A. cannariensis, A. pallens, A. annua, A. abrotanum, A. ludoviciana, A. capillaris und/oder A. scoparia handelt.

9. Verwendung nach einem der Ansprüche 4 bis 8,
**dadurch gekennzeichnet,**
**dass** der Extrakt von einer Subspezies von A. dracunculus stammt.

10. Verwendung nach Anspruch 9,
**dadurch gekennzeichnet,**
**dass** die Subspezies russischer oder französischer Estragon ist.

11. Verwendung nach einem der Ansprüche 1 bis 10,
**dadurch gekennzeichnet,**
**dass** die effektive Menge zwischen 1 und 1,000,000 ppm liegt.

12. Verwendung nach einem der Ansprüche 1-11 zur Behandlung und/oder Vorbeugung von Diabetes Typ 2 und/oder 1 und/oder zur gezielten Beeinflussung des Körpergewichtes.

13. Verwendung einer Zusammensetzung, die gemäß einem der Ansprüche 1 bis 12 definiert ist, zur Steigerung der körperlichen Leistungsfähigkeit.

14. Verwendung nach einem der Ansprüche 1 bis 13,
**dadurch gekennzeichnet,**
**dass** das Mittel als Nahrungsergänzungsmittel, Getränk, Lebensmittel, diätetisches Lebensmittel und/oder Funktionsnahrungsmittel im Rahmen der klinischen Ernährung und/oder als Sportlernahrung eingesetzt wird.

15. Verwendung nach einem der Ansprüche 1 bis 14,
**dadurch gekennzeichnet,**
**dass** das Mittel in Mengen einzusetzen ist, die einer effektiven Tagesmenge der Zusammensetzung, die gemäß einem der Ansprüche 1 bis 11 definiert ist, entspricht und die zwischen 0,1 und 500 mg/kg Körpergewicht liegt.

16. Verwendung nach einem der Ansprüche 1 bis 15,
**dadurch gekennzeichnet,**
**dass** das Mittel neben der physiologisch aktiven Zusammensetzung
a) mindestens eine aktive Verbindung und/oder einen aktiven Extrakt, ausgewählt aus Gymnema sylvestre, Bockshornklee ("Fenugreek"), Bittermelone, α-Liponsäure(-Salze), Corosol-Säure, Ursolinsäure, D-Pinitol, Aloe vera, Chrompicolinat, Banaba, Yacou, Momordica charantia, Olive, Pherocarpus marsupium, Salacia reticulata, Knoblauch, Weißdorn, Phospholipiden, Omega-3-Fettsäuren und/oder Stärke
b) mindestens eine aktive Verbindung, ausgewählt aus Brenztraubensäure, L-Carnitin, Hydroxycitronensäure, Ephedrin, Koffein, konjugierter Linolsäure, Acetylsalicylsäure, α-Liponsäure und/oder deren Salze und/oder
c) mindestens eine aktive Verbindung, ausgewählt aus einer Guanidin-Verbindung, Koffein, α-Liponsäure, Glukosamin, Chondroitin, hydrolysiertes Kollagen, Methylsulfonyl-Methan, Molkeprotein, L-Glutamin, Phospholipiden, sowie Cholin, β-Hydroxy-β-methylbutyrat, Brenztraubensäure, L-Carnitin, D-Ribose, Aminosäuren, S-Adenosylmethionin, Taurin, konjugierter Linolsäure, Glycerin, Zimt und/oder Salze davon
umfasst.

17. Verwendung nach Anspruch 16,
**dadurch gekennzeichnet,**
**dass** im Falle von a) das Phospholipid Phosphatidylserin ist, und im Falle von c) die Guanidin-Verbindung Kreatin, Kreatin-Monohydrat, Guanidinessigsäure, Kreatinol, Kreatin-Citronensäure-Verbindung, Kreatin-pyrovat und/oder Phosphokreatin ist, und das Phospholipid Phosphatidylserin und/oder Phosphatidylcholin ist.

18. Verwendung nach Anspruch 16 oder 17,
**dadurch gekennzeichnet,**
**dass** im Falle von a) das Mittel zur Vorbeugung und/oder Behandlung von (Prä)-Diabetes eingesetzt wird,
im Falle von b) das Mittel zur Körpergewichtskontrolle und insbesondere zur Senkung des Körpergewichts eingesetzt wird, und
im Falle von c) das Mitte zur Steigerung der Zellenenergie in nicht-adiposen Zellen einzusetzen ist.

19. Arzneimittel, umfassend eine physiologisch aktive Zusammensetzung, die gemäß einem der Ansprüche 1 bis 11 definiert ist, und zusätzlich umfassend mindestens eine der in Anspruch 16 genannten aktiven Verbindungen und/oder aktiven Extrakte.

## Claims

1. Use of a physiologically active composition comprising effective amounts of at least one compound selected from 4-O-methyl-davidigenin, 4'-O-methyl-davidigenin, davidigenin, elemicin, isoelemicin, herniarin, demethoxycapillarisine, particularly 6-demethoxycapillarisine and/or 6-demethoxy-3'-methoxycapillarisine, hispiludin and 9-hydroxy-10E, 12Z, 15Z-octadecatrienoic acid as well as their glycosides and additionally at least one compound selected from chlorogen acid, naringenin, flavonoids, flavanoides, terpenes and/or terpenoids for the production of a substance for the prevention and/or treatment of (pre)-diabetes and concomitant and secondary diseases connected with diabetes,
wherein glycosides are selected from the group consisting of glucosides and/or rhamnoglucosides of 4-O-methyldavidigenin and/or 4'-O-methyldavidigenin as well as salts thereof,
and wherein the prevention and/or treatment of (pre)-diabetes and concomitant and secondary diseases connected with diabetes means reducing
a) the blood sugar level in mammals,
b) the insulin resistence,
c) the postprandial glucose level,
and/or rising
d) the expression of IRS-2 ("insulin-receptor-substrate-2")-polypeptide and/or
e) the insulin controlled glucose uptake.

2. Use according to claim 1,
**characterized in that**
it comprises at least one compound in form of glycosides and/or aglycones.

3. Use according to claim 1 or 2,
**characterized in that**
it comprises the compound in pure form and/or as mixture.

4. Use according to claim 3,
**characterized in that**
it comprises the mixture in form of a natural extract.

5. Use according to claim 4,
**characterized in that**
it comprises an alcoholic and/or an alcoholic/aqueous extract.

6. Use according to claim 5,
**characterized in that**
it comprises an ether fraction of the alcoholic/aqueous extract.

7. Use according to any of claims 4 to 6,
**characterized in that**
the extract is an Artemisia extract.

8. Use according to claim 7,
**characterized in that**
the Artemisia extract is an extract of A. dracunculus, A. herba-alba, A. judaica, A. vulgaris, A. abysinica, A. absynthicum, A. afra, A. cannariensis, A. pallens, A. annua, A. abrotanum, A. ludoviciana, A. capillaris and/or A. scoparia.

9. Use according to any of claims 4 to 8,
**characterized in that**
the extract is derived from a subspecies of A. dracunculus.

10. Use according to claim 9,
**characterized in that**
the subspecies is Russian or French tarragon.

11. Use according to any of claims 1 to 10,
**characterized in that**
the effective amount ranges from between 1 and 1.000.000 ppm.

12. Use according to any of claims 1 to 11 for the treatment and/or prevention of diabetes type 2 and/or 1 and/or the specific influence on body weight.

13. Use of a composition defined according to any of claims 1 to 12 for increasing physical efficiency.

14. Use according to any of claims 1 to 13,
**characterized in that**
the substance is applied as dietary supplement, beverage, food, diet food and/or functional food in connection with clinical nutrition and/or as sports food.

15. Use according to any of claims 1 to 14,
**characterized in that**
the substance is to be applied in amounts corresponding to an effective daily amount of the composition defined according to any of claims 1 to 11 and ranging from between 0.1 and 500 mg/kg body weight.

16. Use according to any of claims 1 to 15,
**characterized in that**
the substance comprises, apart from the physiologically active composition
a) at least one active compound and/or an active extract, selected from Gymnema sylvestre, Fenugreek, bitter melon, α-lipoic acid (salts), corosolic acid, ursolic acid, D-pinitol, Aloe vera, chromium picolinate, banaba, yacou, Momordica charantia, olive, Pherocarpus marsupium, Salacia reticulata, garlic, hawthorn, phospholipids, omega-3 fatty acids and/or starch
b) at least one active compound, selected from pyruvic acid, L-carnitine, hydroxycitric acid, ephedrine, coffeine, conjugated linoleic acid, acetylsalicylic acid, α-lipoic acid and/or salts thereof and/or
c) at least one active compound, selected from a guanidine compound, coffeine, α-lipoic acid, glucosamine, chondroitin, hydrolysed collagen, methylsulfonyl-methane, whey protein, L-glutamine, phospholipids, as well as choline, β-hydroxy-β-methylbutyrate, pyruvic acid, L-carnitine, D-ribose, amino acids, S-adenosylmethionine, taurine, conjugated linolic acid, glycerine, cinnamon and/or salts thereof.

17. Use according to claim 16,
**characterized in that**
in case of a) the phospholipid is phosphatidylserine, and in case of c) the guanidine compound is creatine, creatine monohydrate, guanidine acetic acid, creatinole, creatine citric acid compound, creatine pyrovate and/or phosphocreatine, and the phospholipid is phosphatidylserine and/or phosphatidylcholine.

18. Use according to claims 16 or 17,
**characterized in that**
in case of a) the substance is applied for the prevention and/or treatment of (pre)-diabetes,
in case of b) the substance is applied for body weight control and particularly for reducing the body weight, and
in case of c) the substance is to be applied for increasing the cellular energy in non-adipose cells.

19. Medicament comprising a physiologically active composition defined according to any of claims 1 to 11, and additionally comprising at least one of the active compounds and/or active extracts indicated in claim 16.

## Revendications

1. Utilisation d'une composition physiologiquement active comprenant des quantités efficaces d'au moins un composé choisi parmi la 4-O-méthyldavidigénine, la 4'-O-méthyldavidigénine, la davidigénine, l'élémicine, l'isoélémicine, l'herniarine, la déméthoxycapillarisine, en particulier la 6-déméthoxycapillarisine et/ou la 6-déméthoxy-3'-méthoxycapillarisine, l'hispiludine et l'acide 9-hydroxy-10E,12Z, 15Z-octadécatriénoïque, ainsi que leurs glycosides,
et en plus au moins un composé choisi parmi l'acide chlorogénique, la naringénine, les flavonoïdes, les flavanoïdes, les terpènes et/ou les terpénoïdes, pour préparer un agent destiné à la prévention et/ou au traitement du (pré)diabète et de pathologies associées et/ou consécutives au diabète,
dans laquelle les glycosides sont choisis dans le groupe constitué par les glucosides et/ou les rhamnoglucosides de la 4-O-méthyldavidigénine et/ou de la 4'-O-méthyldavidigénine, ainsi que leurs sels, et
dans laquelle la prévention et/ou le traitement du (pré)diabète et de pathologies associées et/ou consécutives au diabète consiste en une diminution
a) de la glycémie chez les mammifères,
b) de la résistance à l'insuline,
c) du taux de glucose postprandial,
et/ou en une augmentation
d) de l'expression du polypeptide IRS-2 (substrat du récepteur à l'insuline 2) et/ou
e) de l'absorption de glucose régulée par l'insuline.

2. Utilisation selon la revendication 1,
**caractérisée en ce**
**qu'**elle comprend au moins un composé sous forme de glycosides et/ou d'aglycones.

3. Utilisation selon la revendication 1 ou la revendication 2,
**caractérisée en ce**
**qu'**elle comprend les composés sous forme pure et/ou sous forme de mélange.

4. Utilisation selon la revendication 3, **caractérisée en ce qu'**elle comprend le mélange sous la forme d'un extrait naturel.

5. Utilisation selon la revendication 4,**caractérisée en ce qu'**elle comprend un extrait alcoolique et/ou hydro-alcoolique.

6. Utilisation selon la revendication 5, **caractérisée en ce qu'**elle comprend une fraction éthérique de l'extrait hydro-alcoolique.

7. Utilisation selon l'une des revendications 4 à 6, **caractérisée en ce qu'**il s'agit d'un extrait d'*Artemisia.*

8. Utilisation selon la revendication 7, **caractérisée en ce que** l'extrait d'*Artemisia* est un extrait d'*A*. *dracunculus, A. herba-alba, A. judaica, A. vulgaris, A. abysinica, A. absynthicum, A. afra, A. canariensis, A. pallens, A. annua, A. abrotanum, A. ludoviciana, A. capillaris* et/ou *A. scoparia.*

9. Utilisation selon l'une des revendications 4 à 8, **caractérisée en ce que** l'extrait provient d'une sous-espèce d'*A*. *dracunculus.*

10. Utilisation selon la revendication 9, **caractérisée en ce que** la sous-espèce est l'estragon de Russie ou l'estragon français.

11. Utilisation selon l'une des revendications 1 à 10, **caractérisée en ce que** la quantité efficace va de 1 à 1 000 000 ppm.

12. Utilisation selon l'une des revendications 1 à 11 pour le traitement et/ou la prévention du diabète de type 2 et/ou de type 1 et/ou pour agir de manière ciblée sur la masse corporelle.

13. Utilisation d'une composition telle que définie dans l'une des revendications 1 à 12 pour augmenter les performances physiques.

14. Utilisation selon l'une des revendications 1 à 13, **caractérisée en ce que** l'agent est utilisé comme complément alimentaire, boisson, aliment, aliment diététique et/ou nutriment fonctionnel dans le cadre de la nutrition clinique et/ou dans l'alimentation des sportifs.

15. Utilisation selon l'une des revendications 1 à 14, **caractérisée en ce que** l'agent doit être utilisés en des quantités correspondant à une quantité journalière efficace de la composition telle que définie dans l'une des revendications 1 à 11 allant de 0,1 à 500 mg par kg de masse corporelle.

16. Utilisation selon l'une des revendications 1 à 15, **caractérisée en ce que**, en plus de la composition physiologiquement active, l'agent contient
a) au moins un composé actif et/ou un extrait actif choisi parmi *Gymnema sylvestre,* la trigonelle (fenugrec), la margose, l'acide α-lipoïque (ou ses sels), l'acide corosolique, l'acide ursolique, le D-pinitol, l'*Aloe vera,* le picolinate de chrome, le banaba, le yacou, *Momordica charantia,* l'olive, *Pherocarpus marsupium, Salacia reticulata,* l'ail, l'aubépine, les phospholipides, les acides gras oméga 3 et/ou l'amidon,
b) au moins un composé actif choisi parmi l'acide pyruvique, la L-carnitine, l'acide hydroxycitrique, l'éphédrine, la caféine, l'acide linoléique conjugué, l'acide acétylsalicylique, l'acide α-lipoïque et/ou leurs sels, et/ou
c) au moins un composé actif choisi parmi un composé de guanidine, la caféine, l'acide α-lipoïque, la glucosamine, la chondroïtine, le collagène hydrolysé, le méthylsulfonylméthane, des protéines du petit-lait, la L-glutamine, des phospholipides, ainsi que la choline, le butyrate de β-hydroxy-β-méthyle, l'acide pyruvique, la L-carnitine, le D-ribose, des acides aminés, la S-adénosylméthionine, la taurine, l'acide linoléique conjugué, le glycérol, la cannelle et/ou des sels de ces substances.

17. Utilisation selon la revendication 16, **caractérisée en ce que** dans le cas de a) le phospholipide est la phosphatidylsérine et dans le cas de c) le composé de guanidine est la créatine, la créatine monohydratée, l'acide guanidinoacétique, le créatinol, le citrate de créatine, le pyruvate de créatine et/ou le phosphocréatine, et le phospholipide est la phosphatidylsérine et/ou la phosphatidylcholine.

18. Utilisation selon la revendication 16 ou la revendication 17, **caractérisée en ce que** dans le cas de a) l'agent est destiné à être utilisé pour la prévention et/ou le traitement du (pré)diabète,
dans le cas de b) l'agent est destiné à être utilisé pour contrôler la masse corporelle et en particulier pour la réduire, et
dans le cas de c) l'agent est destiné à être utilisé pour augmenter l'énergie cellulaire des cellules non adipeuses.

19. Médicament comprenant une composition physiologiquement active telle que définie dans l'une des revendications 1 à 11 et comprenant en plus l'un au moins des composés actifs et/ou extraits actifs cités dans la revendication 16.
